# EUROPEAN PATENT SPECIFICATION

(11) **EP 2 677 945 B1**
(45) Date of publication and mention of the grant of the patent: **19.11.2014**
(21) Application number: 12708601.5
(22) Date of filing: 17.02.2012
(51) Int. Cl.: A61B 17/135

(54) **FEMORAL COMPRESSION DEVICE**
VORRICHTUNG FÜR OBERSCHENKELKOMPRESSION
DISPOSITIF DE COMPRESSION FÉMORALE

(30) Priority: 23.02.2011 SE 1150147; 23.02.2011 US 201161445875 P
(43) Date of publication of application: 01.01.2014
(73) Proprietor: St. Jude Medical Systems AB, 751 35 Uppsala (SE)
(72) Inventor: ADENMARK, Tobias, S-756 46 Uppsala (SE)
(74) Representative: Brann AB
(86) International application number: PCT/SE2012/050173
(87) International publication number: WO 2012/115573

(56) References cited:
- US-A1- 2004 122 469

## Description

### Field of the invention

The present invention relates generally to a femoral compression device and in particular to a femoral compression device comprising a pressurizing means adaptable to varying body constitutions of a patient.

### Background of the Invention

Femoral compression devices for applying pressure on a patient's femoral artery after completion of an interventional procedure are known. An example of such a femoral compression device is disclosed in the patents US 5,307,811 and EP 0 462 088.

A femoral compression device according to these publications comprises basically a pressurizing means for compressive bearing against a puncture site at a femoral artery of a patient, a belt adapted to be fixed around the patient's body, and a base plate supporting the pressurizing means and being provided with two extensions. In use, the pressurizing means, which e.g. has the form of an inflatable semi-spherical air cushion, is positioned over the femoral artery, and the belt, which extends from the end of the first extension, around the patient's body and to the end of the second extension, is tightened. To apply pressure on the femoral artery, the inflatable semi-spherical air cushion is inflated by a pump to a certain predetermined pressure, which is read from a pressure gauge.

The air cushion may be a replaceable air cushion unit preferably packaged in a sterile package to minimize the risk of contamination of a part which is in contact with an area on a patient's body, and which can be exposed to the risk of infection. An example of such a device is disclosed in US 5,542,427, which is assigned to the present applicant. The two part form of appended claim 1 is based on this document. In use, the inflatable air cushion is positioned over a femoral artery of a patient, and the belt, which extends from the end of the first extension, around the patient's body and to the end of the second extension, is tightened. Then, the inflatable air cushion is inflated by a hand pump to a certain internal pressure, thereby expanding the air cushion such that the femoral artery is compressed in order to prevent bleeding through a puncture hole being made in the artery wall. The internal pressure, which can be read from a pressure gauge provided on the pump, should be raised to a value between the diastolic pressure and the systolic pressure - which is a procedure that has proven to work very well for the vast majority of patients. An inherent characteristic of a pneumatic device, and in particular of the inflatable air cushion described above, is that the internal pressure only within a certain operating range corresponds to an increased length of stroke (i.e. increased expansion of the air cushion). For a pressurizing means in the form of an inflatable air cushion, this feature implies that when the air cushion has reached its maximal expansion, a further increase of the internal pressure does not expand the air cushion any more, which, in turn, means that no more compression pressure can be applied on the femoral artery. Normally, i.e. for the vast majority of patients having a normal or ordinary body constitution, this is of no problem since the stroke length (i.e. the expansion) of the air cushion corresponds to the expansion needed to completely, or almost completely, compress the artery such that the flow of blood therethrough is significantly reduced, to thereby prevent bleeding from the puncture wound. In other words, the operating range of the air cushion ranges from a minimum value where the flow of blood is essentially unrestricted to a maximum value where the flow of blood is essentially completely stopped.

However, for those patients where the femoral artery is embedded in a very thick layer of adipose tissue, it can be difficult to determine whether the air cushion has reached its maximal expanded state, in which no more compression of the femoral artery is possible. And when a pressure gauge is used, this problem is even more pronounced because the pressure gauge continues to show increasing values even though the air cushion has reached its maximal expansion. This behavior may give an inexperienced user a deceitful impression that the compression pressure on the femoral artery actually is increasing. Needless to say, such a misjudgment may give rise to very serious complications.

US 5295996 and US 2005/0080440 disclose compression devices with adjustable spacers.

### Summary of the Invention

The above-mentioned problem is solved by the present invention according to the independent claim.

Preferred embodiments are set forth by the dependent claims.

The present invention is therefore directed to an improved femoral compression device, and a general object of the invention is to provide such a compression device for use on patients with a thick layer of adipose tissue at the site of an open puncture in the femoral artery. Embodiments of the invention are directed to a femoral compression device, for use on patients with an excessive amount of adipose tissue in the lower torso region, comprising an inflatable air cushion, a pump, a belt adapted to be fixed around the patient's body, and a base plate supporting the pressurizing means and being provided with two extensions. According to the present invention the inflatable air cushion is raised from the base plate by mounting a spacer between the base plate and the air cushion, and that the spacer has a height which is variable which makes it easy to adapt the femoral compression device to a specific patient. This effectively moves the range of expansion of the air cushion away from the base plate, pressing the excessive adipose tissue to the sides, and ensuring proper access to the compression site. In use the inflatable semi-spherical air cushion is positioned over the femoral artery, and the belt, which extends from the end of the first extension, around the patient's body and to the end of the second extension, is tightened. To apply pressure on the femoral artery, the inflatable semi-spherical air cushion is inflated by a pump effectively compressing the artery such that the flow of blood therethrough is significantly reduced, to thereby prevent bleeding from the puncture wound.

### Brief Description of the Drawings

Figure 1 is a cross-sectional view of a previously proposed femoral compression device attached to the body of a patient having a normal amount of adipose tissue overlying a femoral artery.
Figure 2 is a cross-sectional view of a previously proposed femoral compression device attached to the body of a patient having an excessive amount of adipose tissue overlying a femoral artery.
Figure 3 illustrates a first embodiment of a femoral compression device according to the present invention.
Figures 4a-4d illustrate perspective views of different embodiments of the spacer according to the present invention.
Figure 5 is a cross-sectional view of an alternative embodiment of the spacer.

### Detailed Description of Preferred Embodiments

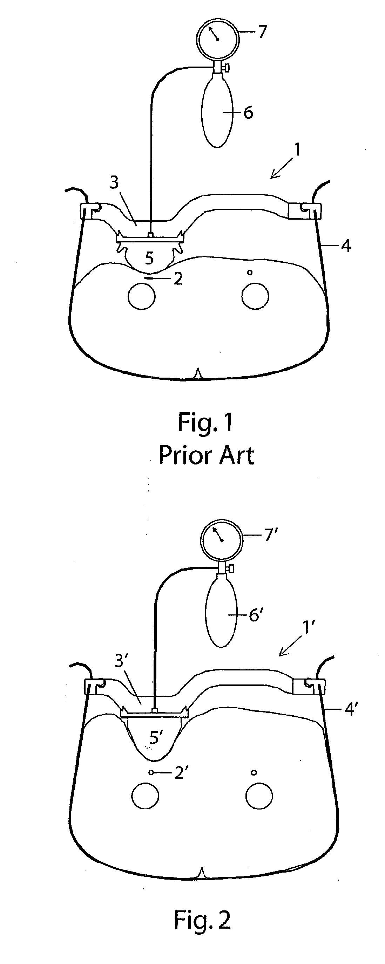

Figure 1 illustrates how a previously proposed femoral compression device 1 is attached to the body of a patient in order to apply compression pressure on a femoral artery 2 in which a puncture hole has been made. The compression device 1 comprises basically a base plate 3, a belt 4 and an inflatable air cushion 5, which can be inflated by a pump 6, which is provided with a pressure gauge 7.

The patient illustrated in figure 1 has a normal body constitution, with an average amount of adipose tissue being localized between the skin and the femoral artery 2. When in a semi-inflated state, i.e. less than fully expanded, the air cushion 5 can therefore compress the artery 2 such that no blood penetrates through the puncture hole in the femoral artery 2. Herein, the expression "normal body constitution" refers to a body constitution to which this existing femoral compression device 1 is adapted, i.e. the stroke length (the expansion) of the air cushion 5 is sufficient for the pressure force being applied therewith to be transmitted through the layer of adipose tissue and compress the femoral artery 2.

Another case is illustrated in figure 2, where a femoral compression device 1' has been attached to the body of an overweight patient to apply compression pressure on a femoral artery 2' in which a puncture hole has been made. The femoral compression device 1' of figure 2 is identical with the femoral compression device 1 shown in fig. 1, and comprises basically a base plate 3', a belt 4' and an inflatable air cushion 5', which can be inflated by a pump 6', which is provided with a pressure gauge 7'. In this case, an excessive amount of adipose tissue is localized between the skin and the femoral artery 2', essentially filling up the space within the boundaries defined by the base plate, the belt and the air cushion. This previously proposed femoral compression device 1' was not designed for this type of patient, and, as is illustrated in the figure, even in the fully expanded state, the air cushion 5' cannot compress the femoral artery 2' enough to stop bleeding through the puncture hole therein. In particular it should be noted that pressure gauges 7 and 7' display the same internal pressure for the two cases illustrated in figure 1 and figure 2, respectively.

As discussed above, a further inflation of the air cushion 5' of Fig. 2 results only in an increase in the internal pressure within the air cushion 5', without any more compression pressure being applied on the femoral artery 2'. Furthermore, the read-out from the pressure gauge 7' will - at least in some sense - support and justify such an operation by the user, because the pressure gauge 7' will continue to show increasing values and thereby give the user the impression that more compression pressure actually is being applied on the femoral artery 2'. In short: when in a not fully expanded state (as in fig. 1), more compression pressure is actually applied on the femoral artery 2 when the air cushion 5 is inflated by the pump 6, which is in accordance with the readings from the pressure gauge 7; whereas in a fully expanded state (as in fig. 2), no more compression pressure is applied on the femoral artery 2' when the air cushion 5' is inflated by the pump 6', which is in contradiction to the readings from the pressure gauge 7'.

In the situation illustrated in figure 2, an inexperienced user may continue to operate the pump 6' in a (vain) attempt to apply more compression pressure on the femoral artery 2', and when the user realizes that the bleeding is not going to stop, there is a risk that the decision will be that the compression device 1' has been misplaced and has to be moved to another position, which leads to unnecessary bleeding. Here it should be mentioned that extra tightening of the belt 4' to some extent could compensate for the above-mentioned disadvantage of the known femoral compression device 1'. However, this procedure requires careful consideration by the user, and the risk of a misleading reading from the pressure gauge 7' is still present. In addition, in those patients where the excess adipose tissue completely or almost completely fills up the space within the boundaries of the base plate, the belt and the air cushion (as illustrated in fig. 2), further tightening of the belt is not possible.

Experience from manual compression (performed by a nurse or other medical practitioner) of obese patients has shown that when initializing the procedure, the person performing the compression needs to penetrate his or her hand deep into the folds of the adipose tissue. According to the present invention a spacer is arranged between the back plate and the air cushion in order to extend the penetration depth of the device into excess adipose tissue, thereby enabling both a considerably improved starting point and working conditions for the procedure.

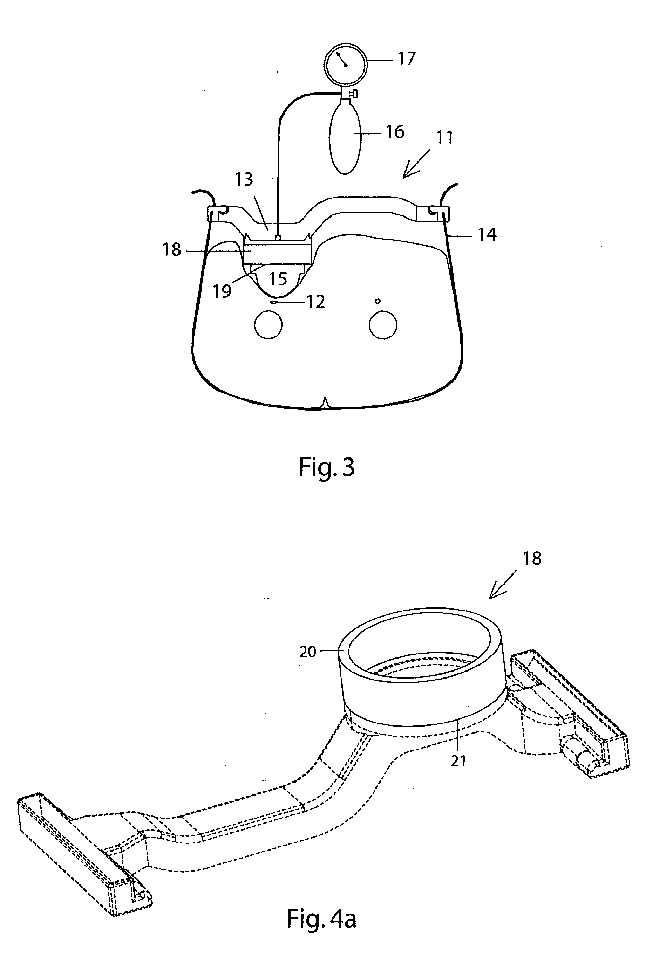

In figure 3 is illustrated a femoral compression device 11 according to the present invention. The device comprises a base plate 13, a belt 14 and an inflatable air cushion 15, which can be inflated by a pump 16, which is provided with a pressure gauge 17. The air cushion is provided with a back plate 19 at the side facing the base plate. Further, the device is provided with a spacer 18 having a height, adapted to be mounted between the inflatable air cushion and the base plate 13. That is achieved by attaching the spacer to the base plate and to the back plate of the air cushion. As seen in figure 3, at the starting point of the procedure, i.e. when the device 11 has been secured to the patient with the belt 14 and the air cushion is yet to be inflated, the excess adipose tissue has been pressed aside due to the extension of the compression component by use of a spacer.

The femoral compression device 11 may be assembled in connection with usage by mounting the spacer to the base plate and then attaching the air cushion to the spacer. The pump is also connected to the air cushion. As an alternative some, or all, parts may be assembled in advance and the femoral compression device, provided with the spacer, is directly ready to be used. This is applicable to all embodiments mentioned herein.

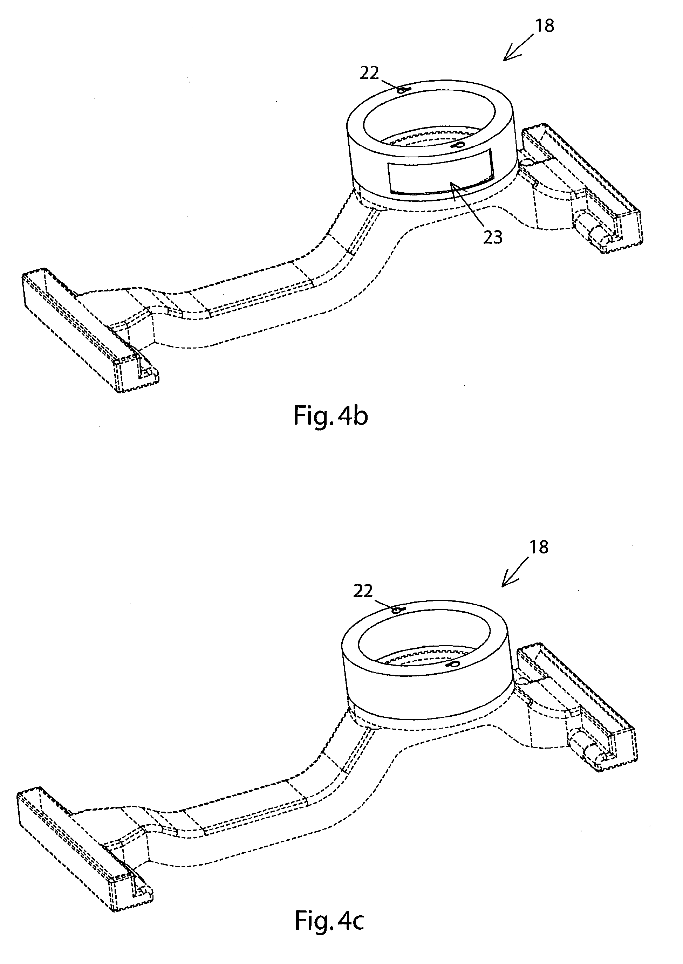

Preferably, the spacer 18 has an essentially circular-cylindrical shape with a cylinder wall having first and second edges 20, 21 (see figure 4a), wherein the first edge 20 is adapted to be attached to the back plate 19 of the air cushion and the second edge 21 is adapted to be attached to the base plate 13.

However, other cross-sectional shapes of the spacer is naturally possible within the scope of the claims as long as the intended purpose of the spacer is achieved, i.e. enabling the air cushion to be arranged at a more distant level in relation to the base plate such that the desired pressure is applied to the femoral artery. The cross-sectional shape may e.g. be elliptical or rectangular as long as it may be attached to the base plate and the back plate of the air cushion.

The spacer is preferably made from a transparent material in order see the wound site when positioning the air cushion in relation to the femoral artery.

For the same purpose the spacer may be provided with at least one opening 23 in the cylinder wall. In addition, the tubing from the pump to the air cushion may be led through the opening.

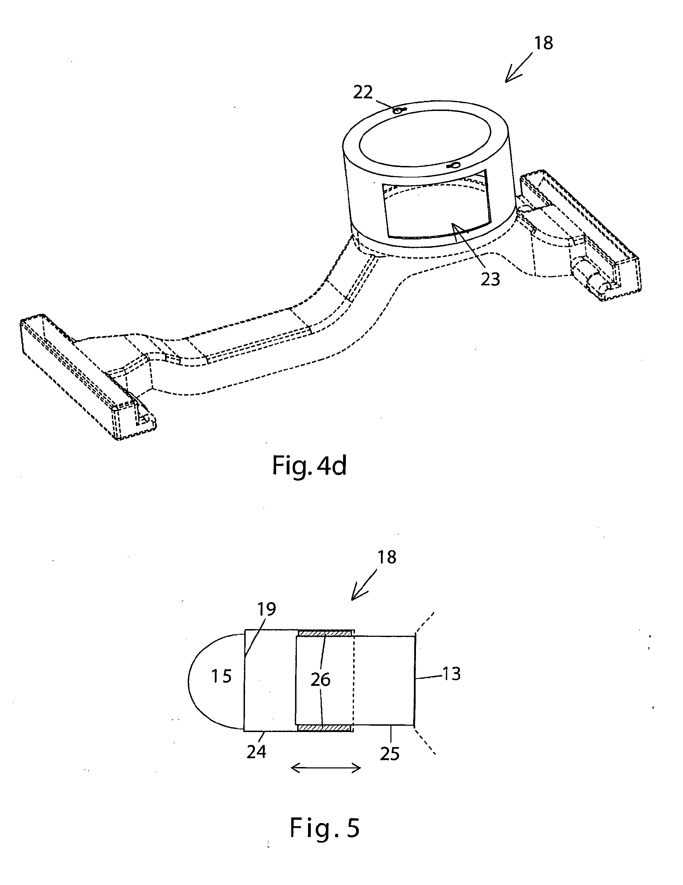

Two spacers having different heights and provided with an opening 23 are illustrated in figures 4b and 4d

The cylinder wall may have a thinner thickness compared to the thickness at the edges which improves the visibility of the wound if the spacer is made from a transparent material.

The first and second edges are each provided with at least one attachment member 22 for cooperation with mating attachment members at the base plate and back plate, respectively. The attachment member 22 may e.g. be a knob and a mating hole which is illustrated in figures 4b-4d.

Alternatively, the attachment members are realized by a bayonet joint.

In still another embodiment the spacer is attached to the base plate and the back plate by an adhesive, e.g. by an self-adhesive at each of the edges that is covered by a plastic foil that is peeled off when the spacer is to be mounted to the base plate and back plate.

The height of the spacer is preferably in the range of 10-100 mm. Even larger heights may be applicable for really obese patients. A set of spacers having different heights may be provided including e.g. five different spacers having heights of e.g. 10, 30, 50, 80 and 100 mm.

In an alternative embodiment the height of the spacer is variable. Figure 5 is a cross-sectional view of a spacer having a variable height. In this embodiment the spacer comprises two concentrically arranged tubes 24, 25 interconnected to each other via internal and external threads 26, such that when the tubes are rotated in relation to each other the height of the spacer may be varied which is indicated in figure 5 by the double-arrow.

According to still another embodiment the spacer comprises two concentrically arranged tubes 24, 25 such that the inner tube 25 is movable in relation to the outer tube 24 and that the spacer is further provided with a locking means adapted to lock the tubes in relation to each other in a desired position. The locking means may e.g. be embodied by an external clamp arranged around the outer tube and adapted to be tightened such that the relative movement is prevented. Alternatively, the locking means may be embodied by at least one insertion pin that is inserted through holes provided in the walls of the tubes at suitable locations in order to insert the pins and thereby fixated the tubes in relation to each other. Still another way to embody the locking means is to arrange a ring-shaped locking means around the inner tube 25 at a desired location chosen to achieve the desired height of the spacer. When the femoral compression device is used a force is present in the direction towards the ring-shaped locking means that thereby prevents the outer tube 24 to move in the direction that lowers the height of the spacer.

Although the present invention has been described with reference to specific embodiments it will be apparent for those skilled in the art that many variations and modifications can be performed within the scope of the invention as described in the specification and defined with reference to the claims below.

## Claims

1. A femoral compression device (11) for compressing a femoral artery (12) of a patient, comprising a base plate (13) provided with two opposing extensions, each of the two opposing extensions comprising a member for affixing a belt (14), which is adapted to be arranged around a patient's body, an inflatable air cushion (15), provided with a back plate (19), for compressive bearing against a puncture site, and a pump (16) connected to the inflatable air cushion for inflation of the air cushion,
**characterized in that** the femoral device further comprises a spacer (18) having a height and being adapted to be mounted between the inflatable air cushion (15) and the base plate (13), thereby defining a spacing distance, wherein said spacing distance is variable, such that the spacing distance may be selected and set by the user before compressing the femoral artery (12).

2. Femoral compression device according to claim 1, wherein said spacer comprises two concentrically arranged tubes (24, 25) adapted to be locked in a desired position by a locking means (26).

3. Femoral compression device according to claim 2, wherein said two concentrically arranged tubes (24, 25) are interconnected to each other via internal and external threads (26), such that when the tubes are rotated in relation to each other the height of the spacer is varied.

4. Femoral compression device according to claim 2, wherein said locking means (26) is a ring-shaped locking means adapted to be arranged around the inner tube 25 at a desired location chosen to achieve the desired height of the spacer.

5. Femoral compression device according to claim 1, wherein said compression device comprises a set of spacers having different heights, and said height is varied by choosing a spacer having a suitable height.

6. Femoral compression device according to any of claims 1-5, wherein said spacer (18) has an essentially circular-cylindrical shape with a cylinder wall having first and second edges (20, 21).

7. Femoral compression device according to claim 6, wherein the first edge (20) is adapted to be attached to the to the back plate (19) of the air cushion (18) and the second edge (21) is adapted to be attached base plate (13).

8. Femoral compression device according to any preceding claim, wherein said spacer is made from a transparent material.

9. Femoral compression device according to any of claims 6-8, wherein said cylinder wall has a thinner thickness compared to the thickness at the edges.

10. Femoral compression device according to any of claims 6-9, wherein said spacer is provided with at least one opening in the cylinder wall.

11. Femoral compression device according to any preceding claim, wherein said height is in the range of 10-100 mm.

## Patentansprüche

1. Eine Oberschenkel-Kompressionseinheit (11) zur Kompression einer Oberschenkelarterie (12) eines Patienten, umfassend eine Basisplatte (13) mit zwei gegenüberliegenden Verlängerungen, wobei jede der zwei gegenüberliegenden Verlängerungen ein Element zur Befestigung eines Bandes (14) umfasst, das zur Anordnung um den Körper des Patienten herum geeignet ist, ein aufblasbares Luftkissen (15), umfassend eine Rückplatte (19), zum Zusammendrücken gegen eine Einstichstelle, und eine Pumpe (16), die zum Aufblasen des aufblasbaren Luftkissens mit dem aufblasbaren Luftkissen verbunden ist,
**dadurch gekennzeichnet, dass** die Oberschenkeleinheit einen Abstandhalter (18) mit einer Höhe umfasst und zur Anbringung zwischen dem aufblasbaren Luftkissen (15) und der Basisplatte (13) angepasst ist, wobei hierdurch ein Abstandsmaß festgelegt ist, wobei das Abstandsmaß derart variabel ist, dass das Abstandsmaß vor dem Komprimieren der Oberschenkelarterie (12) vom Benutzer frei gewählt und festgelegt werden kann.

2. Oberschenkel-Kompressionseinheit nach Anspruch 1, wobei der Abstandhalter zwei konzentrisch angeordnete Röhren (24, 25) umfasst, die angepasst sind, um mittels Arretierungsmittel (26) in der gewünschten Position arretiert zu werden.

3. Oberschenkel-Kompressionseinheit nach Anspruch 2, wobei die zwei konzentrisch angeordneten Röhren (24, 25) über Innen- und Außengewinde (26) derart miteinander verbunden sind, dass sich die Höhe des Abstandhalters ändert, wenn die Röhren im Verhältnis zueinander gedreht werden.

4. Oberschenkel-Kompressionseinheit nach Anspruch 2, wobei das Arretierungsmittel (26) ein ringförmiges Mittel ist, das angepasst ist, um an einer gewünschten Stelle um das Innenrohr 25 angeordnet zu werden, um die gewünschte Höhe des Abstandhalters zu erreichen.

5. Oberschenkel-Kompressionseinheit nach Anspruch 1, wobei die Kompressionseinheit einen Satz von Abstandhaltern mit unterschiedlichen Höhen umfasst und die Höhe durch die Wahl eines Abstandhalters mit einer passenden Höhe variiert wird.

6. Oberschenkel-Kompressionseinheit nach einem der Ansprüche 1-5, wobei der Abstandhalter (18) im Wesentlichen eine kreisförmig-zylindrische Form mit einer Zylinderwand mit ersten und zweiten Kanten (20, 21) aufweist.

7. Oberschenkel-Kompressionseinheit nach Anspruch 6, wobei die erste Kante (20) angepasst ist, um an die Rückplatte (19) des Luftkissens (18) angebracht zu werden, und die zweite Kante angepasst ist, um an die Basisplatte (13) angebracht zu werden.

8. Oberschenkel-Kompressionseinheit nach einem der vorhergehenden Ansprüche, wobei der Abstandhalter aus einem transparenten Material hergestellt ist.

9. Oberschenkel-Kompressionseinheit nach einem der Ansprüche 6-8, wobei die Zylinderwand im Vergleich zur Dicke an den Kanten eine geringere Dicke aufweist.

10. Oberschenkel-Kompressionseinheit nach einem der Ansprüche 6-9, wobei der Abstandhalter mit mindestens einer Öffnung in der Zylinderwand versehen ist.

11. Oberschenkel-Kompressionseinheit nach einem der vorhergehenden Ansprüche, wobei sich die Höhe im Bereich von 10-100 mm befindet.

## Revendications

1. Dispositif de compression fémorale (11) pour comprimer une artère fémorale (12) d'un patient, comprenant une plaque de base (13) pourvue de deux extensions opposées, chacune des deux extensions opposées comprenant un élément pour fixer une courroie (14), qui est adaptée pour être agencée autour du corps d'un patient, un coussin d'air gonflable (15), pourvu d'une plaque arrière (19), pour un appui compressif contre un site de ponction, et une pompe (16) reliée au coussin d'air gonflable pour le gonflage du coussin d'air,
le dispositif fémoral étant **caractérisé en ce qu'**il comprend en outre une entretoise (18) ayant une hauteur et étant adaptée pour être montée entre le coussin d'air gonflable (15) et la plaque de base (13), définissant ainsi une distance d'espacement, ladite distance d'espacement étant variable, de sorte que la distance d'espacement puisse être choisie et réglée par l'utilisateur avant la compression de l'artère fémorale (12)

2. Dispositif de compression fémorale selon la revendication 1, dans lequel ladite entretoise comprend deux tubes agencés de manière concentrique (24, 25) adaptés pour être verrouillés dans une position souhaitée par un moyen de verrouillage (26).

3. Dispositif de compression fémorale selon la revendication 2, dans lequel lesdits deux tubes agencés de manière concentrique (24, 25) sont interconnectés l'un à l'autre par l'intermédiaire de filetages interne et externe (26), de sorte que lorsque les tubes sont mis en rotation l'un par rapport à l'autre la hauteur de l'entretoise varie.

4. Dispositif de compression fémorale selon la revendication 2, dans lequel ledit moyen de verrouillage (26) est un moyen de verrouillage en forme d'anneau adapté pour être agencé autour du tube interne (25) à un emplacement souhaité choisi pour atteindre la hauteur souhaitée de l'entretoise.

5. Dispositif de compression fémorale selon la revendication 1, ledit dispositif de compression comprenant un ensemble d'entretoises ayant des hauteurs différentes, et ladite hauteur varie en choisissant une entretoise ayant une hauteur appropriée.

6. Dispositif de compression fémorale selon l'une des revendications 1 à 5, dans lequel ladite entretoise (18) a une forme essentiellement cylindrique circulaire avec une paroi de cylindre ayant des premier et deuxième bords (20, 21).

7. Dispositif de compression fémorale selon la revendication 6, dans lequel le premier bord (20) est adapté pour être attaché à la plaque arrière (19) du coussin d'air (18) et le deuxième bord (21) est adapté pour être attaché à la plaque de base (13).

8. Dispositif de compression fémorale selon l'une des revendications précédentes, dans lequel ladite entretoise est réalisée en un matériau transparent.

9. Dispositif de compression fémorale selon l'une des revendications 6 à 8, dans lequel ladite paroi de cylindre a une épaisseur plus mince par rapport à l'épaisseur au niveau des bords.

10. Dispositif de compression fémorale selon l'une des revendications 6 à 9, dans lequel ladite entretoise est pourvue d'au moins une ouverture dans la paroi de cylindre.

11. Dispositif de compression fémorale selon l'une des revendications précédentes, dans lequel ladite hauteur est dans la plage allant de 10 à 100 mm.
